# EUROPEAN PATENT APPLICATION

(11) **EP 1 707 961 A2**
(43) Date of publication of application: **04.10.2006**
(21) Application number: 06251807.1
(22) Date of filing: 31.03.2006
(51) Int. Cl.: G01N 33/44, G01N 22/00

(54) **Apparatus for monitoring cure of bone cement**

(30) Priority: 31.03.2005 US 95107
(71) Applicant: DePuy Products, Inc., Warsaw, IN 46581 (US)
(72) Inventor: Sherman, Jason T., Warsaw, IN 46582 (US); DiSilvestro, Mark R., Columbia City, IN 46725 (US)
(74) Representative: Belcher, Simon James

(57) **Abstract**

Apparatus for determining the operating state of a curable bone cement composition includes a microwave transducer (22), which is operated to determine the resonant frequency of the bone cement composition. The resonant frequency is then correlated to the operating state of the bone cement composition.

## Description

The present disclosure relates generally to a technique for monitoring the cure of a bone cement material.

Many orthopaedic procedures require the use of bone cement. Bone cement is used to, for example, secure a prosthetic implant to the patient's natural bone. Most bone cements include a self-curing resin formed from the blending of a liquid monomer or comonomer with a powdered polymer or copolymer. A typical liquid monomer for use as the liquid component of bone cement is a monomeric methyl methacrylate. A typical copolymer powder for use as the powder component of bone cement is a methylmethacrylate-styrene copolymer. Curing of the bone cement composition occurs as the liquid and powder components polymerize and crosslink.

Bone cement is typically mixed in the surgical area just prior to its use. The curing of a bone cement composition is characterized by three operating points. The first of which is dough time. Dough time is distinguished qualitatively as the point in time where the bone cement no longer adheres to latex gloves. Dough time is measured relative to the initial mixing of the liquid and powder components. Dough time signifies the starting point of the working time of the bone cement composition. In other words, once dough time is reached, the bone cement composition has achieved a desired viscosity and flowability to allow for the delivery of the composition into the surgical or implant site.

The end-of-work time is the second operating point of a bone cement composition. It is distinguished qualitatively as the point in time where bone cement no longer adheres to itself. The end-of-work time is also measured relative to the initial mixing of the liquid and powder components. The end-of-work time signifies when the working time of the composition has ended. In other words, the end-of-work time indicates when the bone cement should no longer be used in the surgical procedure.

The third operating point of bone cement is setting time. It, too, is measured relative to the initial mixing of the liquid and powder components. The setting time signifies when the bone cement has cured sufficiently enough to maintain the prosthetic implant in the implant site (for example, in the prepared bone).

The invention provides a method of determining the operating state of a bone cement composition includes determining the resonant frequency of the bone cement composition. The resonant frequency is then correlated to the operating state of the bone cement composition.

Accordingly, in one aspect, the invention provides a method for determining the operating state of a curable bone cement composition, the method comprising the steps of:
exposing the bone cement composition to an electromagnetic field,
determining the resonant frequency of the bone cement composition, and
correlating the resonant frequency to an operating state of the bone cement composition.

In another aspect, the invention provides a method for determining the operating state of a curable bone cement composition, the method comprising the steps of:
determining the resonant frequency of the bone cement composition,
comparing the resonant frequency of the bone cement composition to a predetermined frequency value, and
generating a control signal if the resonant frequency has a predetermined relationship with the frequency value.

The resonant frequency of the bone cement composition may be correlated to dough time, end-of-work time, and setting time of the bone cement composition. The resonant frequency may be used to define a threshold of such times, or may be used as a tool for predicting such times.

A human-detectable signal may be generated when the resonant frequency correlates to a desired operating state of the bone cement composition.

The invention also provides apparatus for determining the operating state of a curable bone cement composition includes an electromagnetic transducer. The electromagnetic transducer is operated to determine the resonant frequency of the bone cement composition. The resonant frequency is then correlated to the operating state of the bone cement composition.

Accordingly, in one aspect, the invention provides apparatus for determining the operating state of a curable bone cement composition, the apparatus comprising:
a bone cement container,
an electromagnetic transducer,
a processor electrically coupled to the electromagnetic transducer, and
a memory device electrically coupled to the processor, wherein the memory device has stored therein a plurality of instructions which, when executed by the processor, cause the processor to:
   operate the electromagnetic transducer to expose a bone cement composition within the container to an electromagnetic field,
   determine the resonant frequency of the bone cement composition within the container, and
   correlate the resonant frequency to an operating state of the bone cement composition.

In another aspect, the invention provides apparatus for determining the operating state of a curable bone cement composition, the apparatus comprising:
an electromagnetic transducer,
a processor electrically coupled to the electromagnetic transducer, and
a memory device electrically coupled to the processor, wherein the memory device has stored therein a plurality of instructions which, when executed by the processor, cause the processor to:
   monitor output from the electromagnetic transducer to determine the resonant frequency of the bone cement composition,
   compare the resonant frequency of the bone cement composition to a predetermined frequency value, and
   generate a control signal if the resonant frequency has a predetermined relationship with the frequency value.

The resonant frequency of the bone cement composition may be correlated to dough time, end-of-work time, and setting time of the bone cement composition. The resonant frequency may be used to define a threshold of such times, or may be used as a tool for predicting such times.

A human-detectable signal may be generated with a visual or audible indicator when the resonant frequency correlates to a desired operating state of the bone cement composition.

Embodiments of the invention will now be described by way of example, with reference to the accompanying drawings, in which:
FIG. 1 is diagrammatic view of an apparatus for monitoring the operating state of a bone cement composition contained in a syringe, note that a portion of the syringe has been cut away for clarity of description;
FIG. 2 is a flowchart of a control routine which is executed by the controller of the apparatus of FIG. 1; and
FIG. 3 is a graph showing a plot of the theoretical relationship between viscosity and resonant frequency of a bone cement composition.

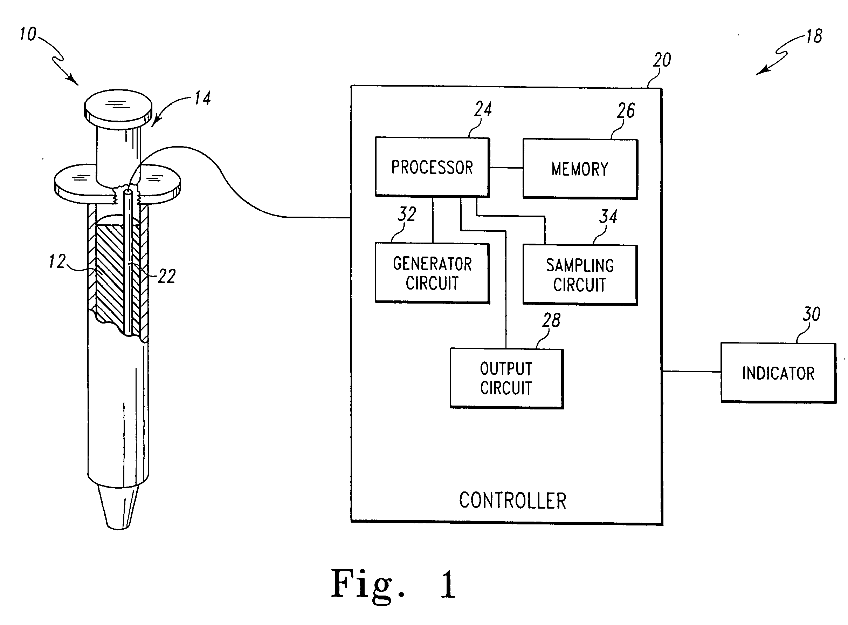

Referring to the drawings, FIG. 1 shows a container 10 having a curable bone cement composition 12 therein. In a known manner, the bone cement composition changes operating states over time as the composition cures. In particular, subsequent to the mixing of the liquid component and the powder component, the physical properties of the bone cement composition change over time. For example, in the case of a bone cement composition of polymethylmethacrylate, the composition polymerizes over time in a manner in which the viscosity or flowability of the composition changes (for example, increases) until the composition is fully hardened.

The container 10 may be embodied as any type of container for containing the bone cement composition 12 as it cures. In the exemplary embodiment of FIG. 1, the container 10 is embodied as a delivery device, such as a syringe 14, for delivering the bone cement composition 12 to a surgical site (for example, the intramedullary canal of the bone into which a prosthesis is being implanted). However, it should be appreciated that the container 10 may be embodied as any other type of container for containing the bone cement composition 12. For example, the container 10 may be embodied as the mixing apparatus (not shown) in which the liquid component and powder component is mixed. The container 10 may be embodied as a test vessel for containing a small sample of the bone cement composition 12. In such a case, subsequent to being mixed, a small portion of the bone cement composition 12 would be placed in the test vessel to be monitored in the manner described below, with the remaining amount of the bone cement composition 12 being used for the surgical procedure.

As shown in FIG. 1, a monitoring apparatus 18 is operated to monitor the operating state of the bone cement composition 12 within the syringe 14 as the composition cures. The monitoring apparatus 18 includes a controller 20 and an electromagnetic transducer 22. The electromagnetic transducer 22 may be operated to generate an electromagnetic field at a desired frequency. In an exemplary embodiment, the transducer 22 may be embodied as a microwave transducer for generating and measuring microwave energy. Microwave frequencies are defined over the range of 100MHz to 300GHz. In a more specific exemplary embodiment, the transducer 22 may be operated to generate and measure microwave energy within the range of 1 to 100GHz.

In the exemplary embodiment of FIG. 1, the transducer 22 is embodied as a microwave radiator in the form of wired antenna which can be positioned within the barrel of the syringe 14 to contact the bone cement composition 12. It should be appreciated, however, that the transducer 22 may be embodied in any configuration of a radiator which fits the needs of a given design. Indeed, the transducer 2 may be embodied in any one of numerous known configurations of a radiator provided that the electromagnetic field generated by the transducer 22 is well coupled to the composition 12. The configuration of the transducer 22 may also be modified to fit the needs of a given type of container 10 in which the bone cement composition 12 is located during polymerization.

The controller 20 includes a processor 24, a memory device 26, and output circuitry 28. The processor 24 may be any type of processor such as, for example, a microprocessor, a microcontroller, or an ASIC. The memory device 26 may be integrated with the processor 24, or may be embodied as a discrete device. The memory device 26 has stored therein the operating and/or application software necessary to operate the monitoring apparatus 18. The output circuitry 28 includes such circuitry commonly found in controllers for controlling and interacting with peripheral devices coupled thereto. For example, the output circuitry 28 includes circuitry for controlling the electromagnetic transducer 22, along with one or more electronically controlled indicators 30.

The indicators 30 may be embodied as a visual indicator and/or an audible indicator. In the case of a visual indicator, one or a series ofLEDs may be used. In such a case, the LED's may be illuminated to represent when the bone cement composition 12 has achieved the three operating states of interest (for example, dough time, end-of-work time, and setting time). In other words, the LED's may be illuminated when the measured resonant frequency of the composition 12 correlates with predetermined empirical values corresponding to the different operating states of the bone cement 12. A tone generator or voice generator may be used to generate an audible indicator in a similar manner.

The controller 20 also includes a field generator circuit 32 that activates the transducer 22 to generate an electromagnetic field, and a sampling circuit 34 that samples the output signal generated by the transducer 22 in response to reflected electromagnetic energy. The sampling circuit may include an analog-to-digital (A/D) converter to convert the output from the transducer 22 into a digital signal that is suitable for presentation to the processor 24.

The monitoring apparatus 18 may be used to determine the viscosity of the bone cement composition 12 within the syringe 14. In particular, by determining the resonant frequency of the bone cement composition 12, the viscosity of the composition 12 may be determined. Specifically, when a dipole, such as the polymerizing bone cement composition 12, is exposed to an electromagnetic field, it attempts to align with the field. When the field is removed, the dipole will relax and return to its original state. The time required for this relaxation varies with the size of the dipole, the strength of the dipole moment, the strength of the applied field, and the media that surrounds it. If a time-varying electromagnetic field is applied, the dipole may be caused to resonate. This occurs when the period (1/f) of the field and the relaxation time are equivalent. There is a direct correlation between the logarithm of viscosity and the logarithm of 1/∈" (with ∈" being the complex component of the dielectric constant). As such, by determining the resonant frequency, the viscosity of the bone cement composition 12 can be determined.

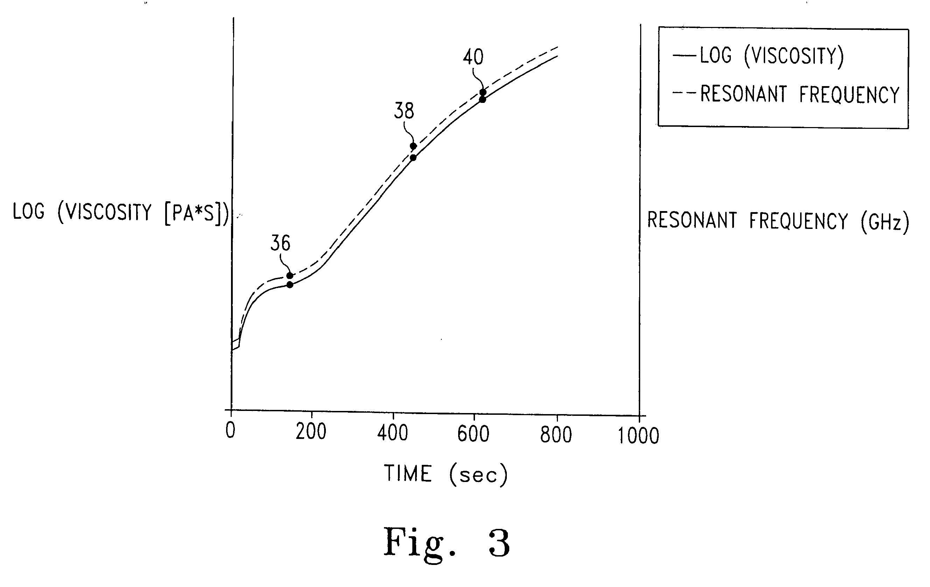

With this, an experimental test may be conducted to gather empirical data relating to, for example, each of the three operating states of interest (for example, dough time, end-of-work time, and setting time) of a particular bone cement composition 12. In such an experimental test, the composition 12 may be exposed to an electromagnetic field during the various stages of polymerization. The frequency of the field may be changed (for example, increased) as a function of time across a range of frequencies, with the response being measured and logged. The frequency at which the highest level of electromagnetic energy is reflected is the resonant frequency (fᵣ). In such a way, the unique resonant frequency of the composition 12 at each operating state may be identified. As such, the resonant frequency of the composition 12 at each of dough time, end-of-work time, and setting time may be empirically determined. A theoretical plot of the results of such an experimental test is shown in FIG. 3. As shown in the graph, both the logarithm of viscosity of a particular bone cement composition and the resonant frequency of the bone cement composition track along somewhat parallel plots over time. The empirical value of dough time is shown graphically on the plots as point 36, whereas end-of-work time and setting time are shown graphically on the plots as points 38 and 40, respectively.

These empirical values may be programmed in the controller 20 for use during a surgical procedure. Specifically, as will be described below in greater detail, during a surgical procedure, the resonant frequency of the bone cement composition 12 may be repeatedly determined (for example, sampled) by the apparatus 12. Such sampled values may then be correlated to the operating state of the bone cement composition 12. For example, each of the sampled resonant frequency values may be compared to the stored empirical values for each of the operating states to determine if the bone cement composition 12 has achieved dough time, end-of-use time, or setting time.

In addition to such threshold correlation of the values, the empirical values may also be used as a predictive tool. For example, by using predictive modelling based upon empirical data collected over a variety of ambient conditions, a "time remaining" estimate could also be calculated. For instance, based on predictive modelling, the resonant frequency of the bone cement composition 12 may be used to determine the amount of time remaining before the bone cement achieves, for example, end-of-work time. Such a tool would be useful for informing the surgeon of the amount of time remaining before the cement delivery phase of the surgical procedure should be completed.

It should be appreciated that one or more of the three operating states of interest (for example, dough time, end-of-work time, and setting time) of a particular bone cement composition 12 may be correlated indirectly from resonant frequency values. For example, in lieu of being correlated directly from a specific resonant frequency value, one or more of the operating states may be correlated from trends in the change of resonant frequency. For example, end-of-work time may not occur at the same frequency under all conditions. However, it may occur at a local minimum frequency under all conditions. Using the first derivate of the resonant frequency would allow for easier identification of such a point. Other indirect correlations between the resonant frequency and the operating states of a bone cement composition, such as correlations to trends in the resonant frequency, may also be used. It should be appreciated that as used herein, the terms "correlate" and "correlating" mean both directly correlate/correlating and indirectly correlate/correlating in regard to the relationship between resonant frequency and the operating states of a bone cement composition.

Moreover, the empirical values may be used in the design of the electronics associated with the controller 22. For example, the controller 22 may be configured to generate and measure three narrow bands of electromagnetic energy (for example., microwave energy), with each band being centred around the empirically determined resonant frequency of each of the three operating states of interest (for example, dough time, end-of-work time, and setting time) of a particular bone cement composition 12.

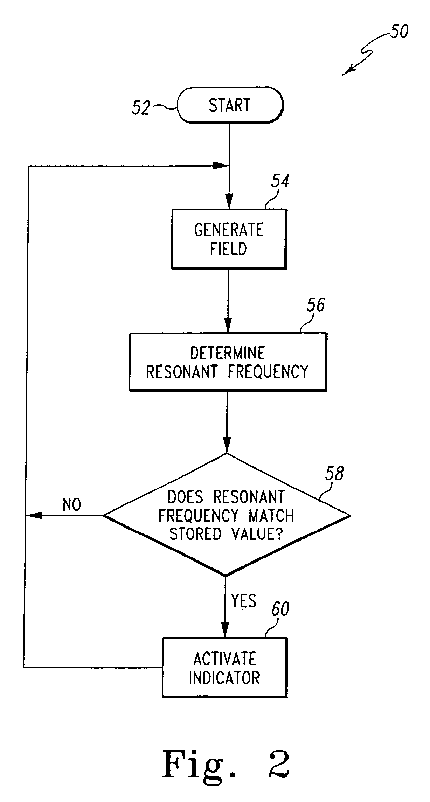

Referring now to FIG. 2, there is shown an exemplary control routine 50 which may be executed by the controller 20 to determine the operating state of the curable bone cement composition 12 during an orthopaedic surgical procedure. Generally, the components of the bone cement composition 12 (i.e., the liquid component and the powder component) are first mixed together. The composition is thereafter monitored as it polymerizes. It should be appreciated that, as described above, the composition may be located in any type of container while it is being monitored. For example, the bone cement composition may be monitored while located in the syringe 14 or a mixing apparatus (not shown). Alternatively, a sample of the composition 12 may be placed in a sample vessel and thereafter monitored from the sample vessel. In any such case, the control routine 50 begins with step 52 in which any variables and/or devices are initialized.

Thereafter, the routine 50 advances to step 54 in which the bone cement composition 12 is exposed to an electromagnetic field. In particular, the processor 24 communicates with the field generator circuit 32 to activate the transducer 22 and generate an electromagnetic field at a desired frequency or range of frequencies. As discussed above, a frequency band may be centred around a predetermined empirical value of a resonant frequency that correlates to a given operating state of interest. The frequency of the electromagnetic field may be scanned through a number of such bands in step 54. Alternatively, the frequency of the electromagnetic field may be scanned through a single, larger frequency band (as opposed to a number of smaller, individual bands) that encompasses each of the empirically generated frequencies. In either case, the field is generated in step 54, and then the routine 50 advances to step 56.

In step 56, the processor 24 determines the resonant frequency of the bone cement composition 12. In particular, the processor 24 monitors output from the sampling circuit 34 which is indicative of the reflected electromagnetic energy (as sensed by the transducer 22). Once the processor 24 has determined the resonant frequency of the composition 12, the routine 50 advance to step 58.

In step 58, the processor 24 correlates the sensed resonant frequency with the operating state of the bone cement composition 12. In an exemplary embodiment, the processor 24 queries a lookup table or other location of the memory device 26 to retrieve the predetermined (for example, empirically created) frequency values associated with the three operating states of interest (for example, dough time, end-of-work time, and setting time) of the particular bone cement composition 12 being sampled. The processor 24 then compares the sensed frequency value to the stored values and determines if the sensed value matches any of the stored values. In other words, the processor 24 compares the sensed resonant frequency value to the predetermined stored values for dough time, end-of-work time, and setting time. If the sensed resonant frequency matches one of the stored values (or is within a predetermined tolerance range of one of the stored values), a control signal is generated and the control routine 50 advances to step 60. If the sensed resonant frequency does not match one of the stored values (or is not within the predetermined tolerance range of one of the stored values), the control routine 50 loops back to step 54 to continue sampling the bone cement composition 12.

In step 60, the processor 24 generates a signal which activates the indicators 30 so as to generate a human-detectable signal. In the case of a visual indicator 30, one or more LED's may be illuminated to represent that the bone cement composition 12 has achieved one of the operating states of interest (for example, dough time, end-of-work time, and setting time). It should be appreciated that the LEDs may be illuminated in a green-yellow-red succession to indicate, respectively, when it is acceptable to use the composition 12 (for example, a green illumination when dough time has been achieved), when the composition 12 is nearing end-of-work time (for example, a yellow illumination when end-of-work time is nearing), and when the composition 12 should no longer be used (for example, a red illumination when end-of-work time has been achieved). In the case of an audible indicator 30, the processor 24 may cause the tone generator or voice generator to generate an audible indication in a similar manner.

After the human-detectable signal has been generated in step 60, the control routine 50 then loops back to step 54 to continue sampling the bone cement composition 12.

In addition to monitoring the curing process, the dielectric analysis techniques described herein may be used to determine information after the bone cement composition has fully set. For instance, the resonant frequency may be tracked after the point of "infinite viscosity" to predict the effective strength of the material.

## Claims

1. Apparatus for determining the operating state of a curable bone cement composition, the apparatus comprising:
a bone cement container,
an electromagnetic transducer,
a processor electrically coupled to the electromagnetic transducer, and
a memory device electrically coupled to the processor, wherein the memory device has stored therein a plurality of instructions which, when executed by the processor, cause the processor to:
operate the electromagnetic transducer to expose a bone cement composition within the container to an electromagnetic field,
determine the resonant frequency of the bone cement composition within the container, and
correlate the resonant frequency to an operating state of the bone cement composition.

2. The apparatus of claim 1, wherein the transducer is configured to generate the electromagnetic field at microwave frequencies.

3. The apparatus of claim 1, wherein the plurality of instructions, when executed by the processor, further cause the processor to generate a control signal if the resonant frequency correlates to a predetermined operating state of the bone cement composition.

4. The apparatus of claim 3, which includes an audible tone generator electrically coupled to the processor, wherein the plurality of instructions, when executed by the processor, further cause the processor to operate the tone generator to generate a human-detectable audible signal in response to generation of the control signal.

5. The apparatus of claim 3, which includes a visual indicator electrically coupled to the processor, wherein the plurality of instructions, when executed by the processor, further cause the processor to operate the visual indicator to generate a human-detectable visual signal in response to generation of the control signal.

6. The apparatus of claim 1, wherein the container comprises a syringe.

7. Apparatus for determining the operating state of a curable bone cement composition, the apparatus comprising:
an electromagnetic transducer,
a processor electrically coupled to the electromagnetic transducer, and
a memory device electrically coupled to the processor, wherein the memory device has stored therein a plurality of instructions which, when executed by the processor, cause the processor to:
monitor output from the electromagnetic transducer to determine the resonant frequency of the bone cement composition,
compare the resonant frequency of the bone cement composition to a predetermined frequency value, and
generate a control signal if the resonant frequency has a predetermined relationship with the frequency value.

8. The apparatus of claim 7, wherein the plurality of instructions, when executed by the processor, further cause the processor to generate the control signal if the resonant frequency matches the frequency value.

9. The method of claim 7, wherein the plurality of instructions, when executed by the processor, further cause the processor to generate the control signal if the resonant frequency is within a predetermined range of the frequency value.

10. The apparatus of claim 7, further comprising an audible tone generator electrically coupled to the processor, wherein the plurality of instructions, when executed by the processor, further cause the processor to operate the tone generator to generate a human-detectable audible signal in response to generation of the control signal.

11. The apparatus of claim 7, which includes a visual indicator electrically coupled to the processor, wherein the plurality of instructions, when executed by the processor, further cause the processor to operate the visual indicator to generate a human-detectable visual signal in response to generation of the control signal.
